**Europäisches Patentamt**

**European Patent Office**

**Office européen des brevets**

(11) Numéro de publication : **0 353 171 B1**

(12) # FASCICULE DE BREVET EUROPEEN

(45) Date de publication du fascicule du brevet :
**25.03.92 Bulletin 92/13**

(51) Int. Cl.⁵ : **A61F 2/34**

(21) Numéro de dépôt : **89420282.9**

(22) Date de dépôt : **27.07.89**

(54) **Cupule de prothèse.**

(30) Priorité : **28.07.88 FR 8810453**

(43) Date de publication de la demande :
**31.01.90 Bulletin 90/05**

(45) Mention de la délivrance du brevet :
**25.03.92 Bulletin 92/13**

(84) Etats contractants désignés :
**BE DE ES FR GB IT NL**

(56) Documents cités :
**EP-A- 0 225 819**
**DE-A- 3 310 944**

(73) Titulaire : **Fayard, Jean-Philippe**
**Le Rochain**
**F-42170 St. Just sur Loire (FR)**
Titulaire : **Gazielly, Dominique**
**47 Rue Henri Dechaud**
**F-42100 Saint Etienne (FR)**
Titulaire : **Passot, Jean-Paul**
**26 Allée des Bois**
**F-42530 St Genest Lerpt (FR)**

Titulaire : **Relave, Marc**
**50 Plein Soleil**
**F-42580 L'Etrat (FR)**
Titulaire : **Roussouly, Pierre**
**41 Rue Boileau**
**F-69006 Lyon (FR)**

(72) Inventeur : **Fayard, Jean-Philippe**
**Le Rochain**
**F-42170 St. Just sur Loire (FR)**
Inventeur : **Gazielly, Dominique**
**47 Rue Henri Dechaud**
**F-42100 Saint Etienne (FR)**
Inventeur : **Passot, Jean-Paul**
**26 Allée des Bois**
**F-42530 St Genest Lerpt (FR)**
Inventeur : **Relave, Marc**
**50 Plein Soleil**
**F-42580 L'Etrat (FR)**
Inventeur : **Roussouly, Pierre**
**41 Rue Boileau**
**F-69006 Lyon (FR)**

(74) Mandataire : **Laurent, Michel et al**
**Cabinet LAURENT et CHARRAS, 20, rue Louis**
**Chirpaz B.P. 32**
**F-69131 Ecully Cedex (FR)**

EP 0 353 171 B1

## Description

L'invention concerne un nouveau type de cupule de prothèse, notamment de prothèse de hanche.

Comme on le sait, une prothèse comprend essentiellement deux parties, à savoir respectivement une tige destinée à être insérée dans l'os de l'articulation à remplacer ou à renforcer, portant à son autre extrémité une tête, dans laquelle vient s'articuler une cupule, elle-même engagée dans l'autre os de l'articulation.

Plus généralement, la cupule est réalisée en deux parties distinctes, à savoir respectivement :
- une cupule dite de "soutien" destinée à être insérée dans l'os de l'articulation ;
- une cupule dite de "frottement" destinée à s'insérer étroitement dans la cupule de maintien, et dont la face interne concave reçoit la tête de la tige de prothèse.

En pratique, la couple de soutien est rigide et est réalisée notamment en métal tel qu'un alliage de titane, alors que la cupule de frottements est le plus généralement réalisée en matière plastique, telle que notamment en polyéthylène.

En pratique, la cupule de soutien est insérée dans l'os selon deux techniques principales. Dans la première technique, la cupule est scellée par des ciments appropriés. Il s'ensuit malheureusement des difficultés de stabilité dans le stabulum, notamment des descellements en cours de portée.

Dans la seconde technique, la cupule est vissée dans l'os de l'articulation à renforcer ou à remplacer. Bien que très largement répandue, cette technique présente l'inconvénient de provoquer parfois des pics de contrainte, qui se traduisent par des douleurs parfois gênantes.

Les caractéristiques du préambule de la revendication 1 sont connues du document EP-A-225 819. Ce document décrit une cupule métallique sphérique pour prothèse de hanche non cimentée, formée en deux zones, la première en calotte sphérique décalée, portant deux chevilles cylindriques parallèles pour le passage et le guidage des vis d'ancrage primaires dans le matériau osseux spongieux, la seconde comportant des fentes de longueur décroissante disposées suivant des méridiens. La première zone est garnie d'un revêtement de titane poreux pour assurer un ancrage secondaire, alors que la seconde zone est lisse. Du fait de la longueur décroissante des fentes méridiennes, la cupule réalisée de la sorte ne présente pas la même élasticité de déformation dans tous les secteurs. Il s'ensuit que la tenue de l'ensemble dans l'os est essentiellement assurée par les vis d'ancrage. On est donc ainsi tributaire de la qualité du matériau osseux. Enfin, la mise en place et le blocage de la cupule de frottement est assurée par clipsage-emboitement mécanique, grâce notamment à un ensemble téton-échancrure pour assurer le blocage en rotation.

Dans le document DE-A-3 310 944, on a suggéré de fendre les chevilles lisses à leurs extrémités pour assurer une certaine expansion lors du vissage. Toutefois, du fait de la longueur très réduite de cette fente et de la surface externe lisse, l'expansion ainsi réalisée donc l'accrochage est très limité.

L'invention pallie ces inconvénients. Elle vise une cupule du type en question, c'est-à-dire comportant deux cupules insérées l'une dans l'autre, respectivement de soutien, et de frottement, qui soit facile et économique à réaliser, facile à mettre en place et à retirer, et ne présente pas les inconvénients rappelés ci-dessus, notamment en cours de portée.

L'invention vise à réaliser une cupule perfectionnée du type en question qui assure un meilleur blocage dans l'os, une excellente tenue dans le temps et une bonne stabilité primaire et secondaire.

La cupule de prothèse selon l'invention, du type comprenant :
- une cupule dite "de soutien" destinée à être insérée dans l'os de l'articulation à renforcer ou à remplacer, et qui présente sur sa face convexe externe d'une part, au moins une cheville expansible et, d'autre part, une pluralité de fentes d'expansion disposées sur des cercles passant par le pôle et ouvertes sur l'équateur ;
- une cupule dite "de frottement", en matière plastique, destiné à s'insérer étroitement dans la cupule de soutien, et dont la face interne concave, destinée à recevoir la tête de la tige de prothèse, est hémisphérique,

**se caractérise :**
- en ce que l'une des fentes d'expansion est prolongée jusqu'au pôle de la cupule de soutien où elle débouche dans un arrondi ;
- et en ce que la base de la face interne de la cupule de soutien comporte un filetage destiné à coopérer avec un filetage complémentaire ménagé à cet effet sur la face externe de la cupule de frottement dans la portion proche de l'équateur.

En d'autres termes, l'invention consiste à ménager une fente de la cupule de soutien jusqu'au pôle pour assurer une bonne élasticité à l'ensemble et à réaliser l'expansion de cette cupule par le vissage même de la cupule de frottement dans celle-ci.

Ainsi, l'invention permet d'insérer la cupule par expansion entre les deux cornes du cotyle antérieure et postérieure, d'une part grâce aux chevilles expansibles, et d'autre part, grâce aux fentes de la cupule de soutien qui sont expansées lors de la mise en place de la cupule de frottement. Ainsi, la mise en place de la cupule de frottement participe à l'expansion de la cupule de soutien et ce, grâce aux fentes, ce qui se traduit par un meilleur accrochage dans le matériau osseux. Grâce à la longueur sensiblement également des fentes méridiennes, la cupule peut être positionnée de manière très diverse dans les cornes du cotyle.

Avantageusement, en pratique :
– on fait appel à deux chevilles expansibles symétriques ;
– les chevilles expansibles d'une part et les fentes d'expansion d'autre part, sont disposées respectivement sur chacune de deux portions opposées respectivement de la face droite et de la face gauche de la cupule de soutien ;
– les chevilles expansibles sont rainurées à l'extérieur en forme de harpons et sont fendues axialement sur partie essentielle de leur longueur, de manière à définir au moins deux portions symétriques susceptibles d'être écartées l'une de l'autre sous l'effet d'un coin en vis inséré entre ces deux portions ;
– le coin d'écartement est consitué par une vis actionnée depuis la face interne de la cupule de soutien;
– les fentes d'expansion de la cupule définissent des secteurs égaux en forme de corolle ;
– la fente prolongée jusqu'au pôle est la fente médiane ;
– la cupule de soutien est en un alliage de titane, notamment du type TA6V, et la cupule de frottement est en polyéthylène haute densité ;
– la surface externe de la cupule métallique de soutien est grenaillée.

La manière dont l'invention peut être réalisée et les avantages qui en découlent ressortiront mieux de l'exemple de réalisation qui suit à l'appui des figures annexées.

La figure 1 montre une vue en coupe d'une cupule dépouillée, conforme à l'invention.

La figure 2 est une vue de dessus de la cupule de soutien caractéristique.

La figure 3 est une représentation schématique sommaire en coupe longitudinale d'une cupule conforme à l'invention, dans laquelle les chevilles expansibles vissés sont en position normale, alors qu'ils sont en position écartée à la figure 4.

La figure 5 montre une vue détaillée de l'architecture externe des chevilles expansibles conformes à l'invention.

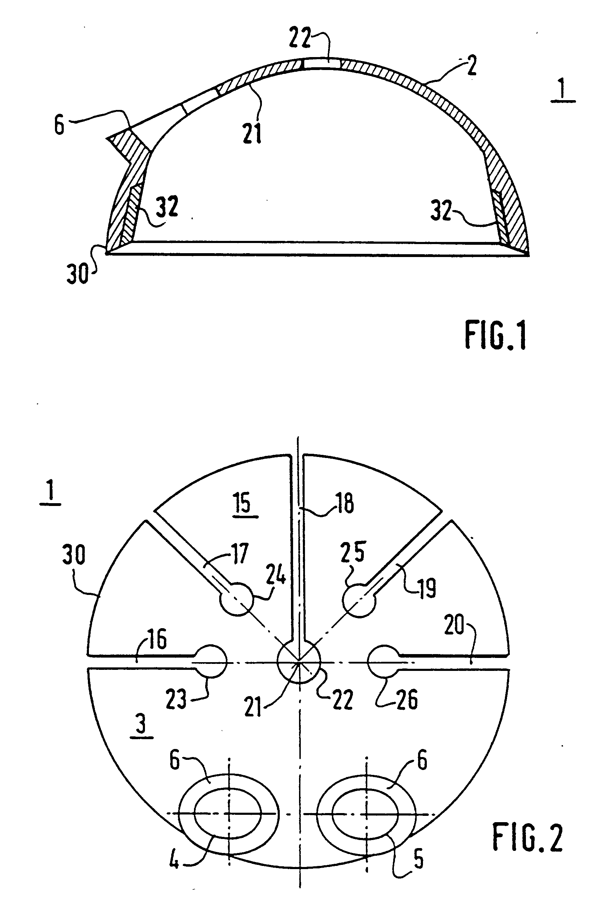

La cupule de soutien conforme à l'invention, représentée aux figures 1 et 2 a une forme générale hémisphérique et est réalisée dans un alliage de titane, notamment du type TA6V (titane + aluminium 6 % + vanadium 4 %). La surface extérieure (2) de cette cupule de soutien désignée par la référence générale (1) est grenaillée, de manière à obtenir un état de surface poreux approprié, présentant une pluralité de porosités ouvertes, par exemple ayant des ouvertures de cinquante à cent micromètres, de préférence au voisinage de quatre-vingt micromètres. Comme on le sait, ces état de surface poreux favorise la réhabilitation osseuse.

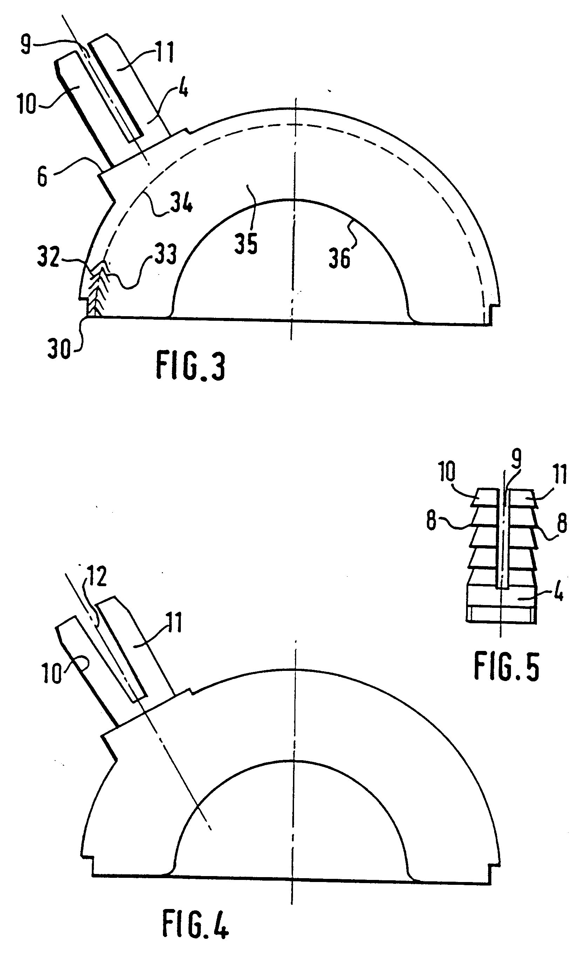

Selon l'invention, cette cupule de soutien (1) présente sur sa face externe (2) d'une part, sur la première portion (3), deux chevilles symétriques respectivement (4) et (5), montrées en détail à la figure 5, vissées dans une embase (6) prévue à cet effet. Comme on peut le voir sur la figure 5, chaque cheville (4,5) est expansible. Pour ce faire, chaque cheville (4,5) présente à l'extérieur une pluralité de rainures successives (8) en forme de harpon pour former retenue et est fendu axialement (9) sur partie essentielle de sa hauteur pour définir deux ou quatre portions symétriques (10) et (11), susceptibles d'être écartées l'une de l'autre sous l'effet d'un coin (12) (figure 4), inséré entre ces portions (10,11). Ces chevilles expansibles (4,5) qui sont orientées suivant l'axe des forces maximales, participent de manière prépondérante à la stabilité primaire dans le cotyle. En pratique, le coin est une vis (12) introduite dans les fentes (9) depuis l'intérieur (31).

La seconde portion (15) de la cupule de soutien (1), opposée à (3), comporte selon une seconde caractéristique de l'invention, une pluralité de fentes d'expansion en l'occurrence (16,17,18,19,20), disposées sur des cercles méridiens imaginaires passant par le pôle (21) et ouvertes sur l'équateur (30). Ces fentes d'expansion (16-20) de même longueur sont équidistantes et définissent ainsi des secteurs égaux en forme de corolle. Selon une autre caractéristique importante de l'invention, la fente médiane (18) est prolongée jusqu'au pôle (21) en débouchant sur un arrondi (22) disposé au sommet de la cupule hémisphérique de soutien (1). Les autres fentes (16,17,19,20) de même longueur débouchent également sur des arrondis (23-26) alignés sur une calotte. La fente médiane (18) qui débouche au pôle (21) donne à l'ensemble une meilleure élasticité. Les arrondis (22-26) permettent avantageusement d'introduire des petits greffons pour favoriser la reprise osseuse. La disposition symétrique des fentes (16-20) de même longueur confère la même élasticité aux différents secteurs en corolle, ce qui permet d'assurer un meilleur positionnement (ce que ne permet pas d'obtenir la réalisation décrite dans le brevet EP-O-225 819 visé dans le préambule, dans lequel les fentes ont une longueur décroissante).

Selon une autre caractéristique importante de l'invention (voir figures 1 et 3), la base (30) de la face interne (31) de la cupule de soutien (1) présente un filetage (32) destiné à coopérer avec un filetage complémentaire (33) ménagé à cet effet à la base de la face externe (34) de la cupule de frottement (35) dans la portion proche de l'équateur (30). De manière connue, la cupule de frottement (35) est en polyéthylène haute densité.

En pratique, la forme et les côtes de la face externe (34) de la cupule de frottement (35) sont légèrement supérieures à celles de la face interne (31) complémentaire de la cupule de soutien (1), du moins au voisinage de l'équateur (30). Il suffit par exemple que le diamètre externe de la cupule de frottements (35) au niveau du filetage (33) ait de l'ordre de un à deux millimètres de plus que le diamètre interne du filetage (32) disposé à la base (30) de la face interne (31) de la cupule de soutien (1). Il s'ensuit qu'en vissant la cupule de frottements (35) dans la cupule de soutien (1), on expanse progressivement les corolles formées par les fentes (16-20) qui débouchent sur l'équateur (30). Grâce à cette coopération et interaction entre les fentes (16-20) et la cupule de frottement (35), on améliore la mise en place et on assure une bonne stabilité à l'ensemble, tout en facilitant l'expansion de la cupule de frottement (35) dans la cupule de soutien (1).

La cupule selon l'invention est mise en place de la manière suivante.

De manière connue, le chirurgien fraise le cotyle à la dimension de la cupule hémisphérique de soutien (1) choisie. A l'aide d'un gabarit, il perce deux trous parallèles au moyen d'un foret souple à butée, dans l'axe des chevilles (4,5) expansibles, ces orifices étant disposés à 30°. Il impacte la cupule (1) de soutien dans le logement ainsi taillé. En insérant dans les fentes longitudinales (9) des vis, il expanse les chevilles (4,5) (voir figure 4), ce qui grâce aux harpons (8) assure une bonne stabilité primaire dans le matériau spongieux.

Il insère ensuite la cupule de frottement (35) en la vissant (32,33) dans la cupule de soutien (1). De la sorte, il solidarise, d'une part, la cupule de soutien (1) avec la cupule de frottement (35) qui, en fin de course, expanse progressivement la cupule de soutien (1) en écartant les corolles élémentaires formées par les fentes successives équidistantes (16-20) contre les cornes antérieures et postérieures du cotyle pour assurer une meilleure tenue.

L'invention se distingue essentiellement de la technique décrite dans le document EP-A-0 225 819 cité dans le préambule par, d'une part, la présence des fentes méridiennes de longueur constante, ce qui permet d'assurer une pluralité de positionnements de la cupule, d'autre part, par le fait que la fente médiane (18) débouche au pôle (21), ce qui confère une meilleure élasticité à la cupule de soutien (1) lors de l'expansion, par ailleurs, par le fait que les fentes (16-20) sont terminées par des arrondis (22-26), ce qui permet d'introduire des greffons, et enfin, par la présence de chevilles expansibles qui assurent une bonne stabilité primaire et pour terminer, par la coopération avec la cupule de frottement (35) qui, lors du vissage, participe à l'expansion des corolles formées par les fentes méridiennes (16-20).

L'invention se distingue essentiellement du document DE-A-3310944 cité dans le préambule par le fait que la cupule de soutien (1) est expansible, et par le fait que les chevilles (4,5) sont expansibles sur partie appréciable de leur longueur et présentent un état de surface irrégulier qui augmente la prise donc la tenue dans le matériau spongieux.

La cupule selon l'invention présente de nombreux avantages par rapport à celles commercialisées à ce jour ou citées dans le préambule. On peut citer :
– une excellente stabilité primaire immédiate ;
– une facilité de mise en oeuvre ;
– la possibilité d'insérer des greffons sous la cupule de soutien pour faciliter la reprise osseuse ;
– l'absence de vissage dans l'os, donc de pics de contrainte ;
– une très bonne facilité de pose et de dépose ;
– l'interchangeabilité des pièces.

De la sorte, cette cupule peut être utilisée avec succès dans de nombreuses prothèses, notamment les prothèses de hanche.

## Revendications

1. Cupule de prothèse, du type comprenant :
– une cupule (1) dite de "soutien" destinée à être insérée dans l'os de l'articulation à renforcer ou à remplacer et qui présente sur sa face convexe externe, d'une part, au moins une cheville (4,5) et, d'autre part, une pluralité de fentes (16-20) d'expansion disposées sur des cercles méridiens passant par le pôle (21) et ouvertes sur l'équateur (30),
– une cupule (35) dite de "frottement", en matière plastique, destinée à s'insérer étroitement dans la cupule de soutien (1) dont la face interne concave (36) destinée à recevoir la tête de la tige de prothèse est hémisphérique,
**caractérisée** :
– en ce que la cheville (4,5) est expansible ;
– en ce que l'une des fentes (16-20) d'expansion est prolongée jusqu'au pôle (21) de la cupule de soutien (1) où elle débouche dans un arrondi (22) ;
– et en ce que la base (30) de la face interne (31) de la cupule de soutien (1) comporte un filetage (32) destiné à coopérer avec un filetage complémentaire (33) ménagé à cet effet à la base de la face externe (34) de la cupule de frottement (35), dans la portion proche de l'équateur (30).

2. Cupule selon la revendication 1, **caractérisée** en ce que la cupule de soutien est en métal, en ce que la forme de la face externe (34) de la cupule de frottements (33) correspond à celle de la face interne (31) de la cupule de soutien (1), et en ce que le diamètre de la portion filetée (33) de la base de la cupule de frottement (35) est légèrement supérieur au diamètre interne du filetage (32) disposé à la base (30) de la face interne (31) de la cupule de soutien (1).

3. Cupule selon la revendication 1, caractérisée en ce que les chevilles expansibles (4,5), d'une part, et les fentes d'expansion (16-20) d'autre part, sont disposés respectivement sur chacune de deux portions symétriques (3,15) opposées, respectivement de la face droite (3) et de la face gauche (15) de la cupule de soutien (1).

4. Cupule selon l'une des revendications 1 à 3, caractérisée en ce que les chevilles expansibles (4,5) sont rainurés à l'extérieur en forme de harpons (8) et sont fendus axialement (9) sur partie essentielle de leur longeur, de manière à définir au moins deux portions symétriques (10,11) susceptibles d'être écartées l'une de l'autre sous l'effet d'un coin (12) inséré entre ces deux portions écartables (10,11).

5. Cupule selon la revendication 4, caractérisée en ce que le moyen d'écartement des deux portions écartables (10,11) est constitué par une vis (12) actionnée depuis la face interne (31) de la cupule de soutien (1).

6. Cupule selon l'une des revendications 3 à 5, caractérisée en ce qu'elle comporte deux chevilles expansibles (4,5) symétriques.

7. Cupule selon l'une des revendications 1 à 6, caractérisée en ce que les fentes d'expansion (16,17,19-20) de même longueur sont équidistantes et définissent entre elles des secteurs égaux en forme de corolle, à l'exception de la fente médiane (18) qui est prolongée jusqu'au pôle (21) pour se terminer par un arrondi (22).

8. Cupule selon la revendication 7, caractérisée en ce que les fentes d'expansion (16,17,19,20) de même longueur ouvertes sur l'équateur (30) se terminent par un arrondi (23-26).

9. Cupule selon la revendication 2, caractérisée en ce que la cupule de soutien (1) est en un alliage de titane, alors que la cupule de frottement (35) est en polyéthylène haute densité.

10. Cupule selon l'une des revendications 1 à 9 caractérisée en ce que la surface externe de la cupule de soutien (1) et des chevilles expansibles (4,5) est grenaillée.

**Patentansprüche**

1. Prothetische Gelenkpfanne, enthaltend:
– eine Stützpfanne (1), die dazu vorgesehen ist, in den zu verstärkenden oder zu ersetzenden Gelenkknochen eingesetzt zu werden und die auf ihrer äußeren konvexen Fläche einerseits zumindest einen Zapfen (4, 5) und andererseits eine Vielzahl an Dehnungsspalten (6-20) aufweist, die entlang von Meridiankreisen angeordnet sind, die durch den Pol (21) verlaufen und zum Äquator (30) hin öffnen,
– eine Reibpfanne (35) aus Kunststoffmaterial, die dazu vorgesehen ist, engsitzend in die Stützpfanne (1) eingebracht zu werden, wobei deren innere konkave Fläche (36), die dazu vorgesehen ist, den Kopf des Prothesenschaftes aufzunehmen, halbkugelförmig ist, dadurch gekennzeichnet,
– daß der Zapfen (4, 5) aufweitbar ist;
– daß einer der Dehnungsspalte (16-20) bis zum Pol (21) der Stützpfanne (1) verlängert ist, wo er in einer Rundung (22) mündet;
– und daß die Basis (30) der Innenfläche (31) der Stützpfanne (1) ein Gewinde (32) aufweist, das dazu vorgesehen ist, mit einem entsprechenden Gewinde (33) zusammenzuwirken, das auf der Außenfläche (34) der Reibpfanne (35) in der Nähe des Äquators (30) ausgespart ist.

2. Gelenkpfanne nach Anspruch 1, dadurch gekennzeichnet, daß die Stützpfanne aus Metall besteht, daß die Form der Außenfläche (34) der Reibpfanne (35) der Innenfläche (31) der Stützpfanne (1) entspricht, und daß der Durchmesser des mit dem Gewinde versehenen Bereiches (33) der Basis der Reibpfanne (35) etwas größer ist als der Innendurchmesser des Gewindes (32), das an der Basis (30) der Innenfläche (31) der Stützpfanne (1) vorgesehen ist.

3. Gelenkpfanne nach Anspruch 1, dadurch gekennzeichnet, daß die aufweitbaren Zapfen (4, 5) einerseits und die Dehnungsspalte (16-20) andererseits jeweils auf einem von zwei gegenüberliegenden symmetrischen Abschnitten (3, 15), nämlich der rechten Seite (3) und der linken Seite (15) der Stützpfanne (1) angeordnet sind.

4. Gelenkpfanne nach einem der Ansprüche 1 bis 3, dadurch gekennzeichnet, daß die aufweitbaren Zapfen (4, 5) an ihrer Außenseite hakenförmig (8) gerillt sind, und über den größten Teil ihrer Länge axial gespalten (9) sind, so daß zumindest zwei symmetrische Abschnitte (10, 11) gebildet sind, die unter der Wirkung eines Keiles (12), der zwischen die beiden aufweitbaren Abschnitte (10, 11) eingeschoben wird, gespreizt werden können.

5. Gelenkpfanne nach Anspruch 4, dadurch gekennzeichnet, daß die Mittel zum Aufspreizen der beiden aufweitbaren Abschnitte (11, 12) durch eine Schraube (12) gebildet werden, die von der Innenseite (31) der Stützpfanne (1) her betätigbar ist.

6. Gelenkpfanne nach einem der Ansprüche 3 bis 5, dadurch gekennzeichnet, daß sie symmetrisch zwei aufweitbare Zapfen (4, 5) aufweist.

7. Gelenkpfanne nach einem der Ansprüche 1 bis 6, dadurch gekennzeichnet, daß die Dehnungsspalte (16, 17, 19-20) gleicher Länge gleichmäßig voneinander beabstandet sind und zwischen sich gleiche kronenförmige Abschnitte umgrenzen, mit Ausnahme des medianen Dehnungsspaltes (18), der bis zum Pol (21) verlängert ist, wo er in einer Rundung (22) endet.

8. Gelenkpfanne nach Anspruch 7, dadurch gekennzeichnet, daß die sich zum Äquator (30) öffnenden Dehnungsspalte (16, 17 19, 20) gleicher Länge in einer Rundung (23-26) enden.

9. Gelenkpfanne nach Anspruch 2, dadurch gekennzeichnet, daß die Stützpfanne (1) aus einer Titanlegierung besteht, wohingegen die Reibpfanne (35) aus Polyethylen hoher Dichte besteht.

10. Gelenkpfanne nach einem der Ansprüche 1 bis 9, dadurch gekennzeichnet, daß die Außenfläche der Stützpfanne (1) und der spreizbaren Zapfen (4, 5) körnig ist.

## Claims

1. Prosthetic cupule, of the type comprising :
– a cupule (1) called a "support cupule" which is intended to be inserted in the bone of the joint to be strengthened or to be replaced, and which has on its outer convex face, on the one hand, at least one plug (4, 5) and, on the other hand, a plurality of expansion slots (16-20) arranged on meridian circles passing through the pole (21) and open on the equator (30),
– a cupule (35) called a "friction cupule, made of plastic material and intended to be inserted tightly into the support cupule (1), and of which the concave inner face (36) intended to receive the head of the prosthetic stem is semi-circular,
characterised:
– in that the plug (4, 5) is expandable;
– in that one of the expansion slots (16-20) extends as far as the pole (21) of the support cupule (1) where it opens into a rounded area (22);
– and in that the base (30) of the inner face (31) of the support cupule (1) comprises a thread (32) intended to co-operate with a complementary thread (33) made for this purpose at the base of the outer face (34) of the friction cupule (35), in the portion near the equator (30).

2. Cupule according to Claim 1, characterised in that the support cupule is made of metal, in that the shape of the outer face (34) of the friction cupule (35) corresponds to that of the inner face (31) of the support cupule (1), and in that the diameter of the threaded portion (33) of the base of the friction cupule (35) is slightly greater than the internal diameter of the thread (32) arranged at the base (30) of the inner face (31) of the support cupule (1).

3. Cupule according to Claim 1, characterised in that the expandable plugs (4, 5) on the one hand and the expansion slots (16-20) on the other hand are arranged, respectively, on each of two opposite symmetrical portions (3, 15), respectively, of the right-hand face (3) and of the left-hand face (15) of the support cupule (1).

4. Cupule according to one of Claims 1 to 3, characterised in that the expandable plugs (4, 5) are grooved on the outside in the shape of harpoons (8) and are slotted axially (9) over the greater part of their length, in such a way as to define at least two symmetrical portions (10, 11) capable of being spaced apart one from the other under the effect of a wedge (12) inserted between these two separable portions (10, 11).

5. Cupule according to Claim 4, characterised in that the means for spacing apart the two separable portions (10, 11) consists of a screw (12) activated from the inner face (31) of the support cupule (1).

6. Cupule according to one of Claims 3 to 5, characterised in that it comprises two symmetrical expandable plugs (4, 5).

7. Cupule according to one of Claims 1 to 6, characterised in that the expansion slots (16, 17, 19, 20) of identical length are equidistant and define between them equal sectors of corolla shape, with the exception of the median slot (18) which extends as far as the pole (21) and terminates in a rounded area (22).

8. Cupule according to Claim 7, characterised in that the expansion slots (16, 17, 19, 20) of identical length and open on the equator (30) end in a rounded area (23-26).

9. Cupule according to Claim 2, characterised in that the support cupule (1) is made of a titanium alloy, while the friction cupule (35) is made of high-density polyethylene.

10. Cupule according to one of Claims 1 to 9, characterised in that the outer surface of the support cupule (1) and of the expandable plugs (4, 5) is grained.

**22**

**2**

**6**

**21**

**32**

**32**

**30**

**FIG.1**

**1**

**1**

**30**

**15**

**18**

**17**

**25**

**24**

**16**

**19**

**20**

**23**

**21**

**22**

**26**

**3**

**6**

**6**

**4**

**5**

**FIG.2**

FIG.3

FIG.5

FIG.4